(19) Europäisches Patentamt
European Patent Office
Office européen des brevets

(11) Numéro de publication: **0 141 266**
**B1**

(12) **FASCICULE DE BREVET EUROPEEN**

(45) Date de publication du fascicule du brevet: **07.12.88**

(51) Int. Cl.[4]: **A 61 L 9/04,** A 61 L 9/01

(21) Numéro de dépôt: **84111620.5**

(22) Date de dépôt: **28.09.84**

(54) **Utilisation de l'éther méthylique de l'alpha-terpényle à titre d'adjuvant à des compositions assainissantes parfumées.**

(30) Priorité: **21.10.83 CH 5730/83**

(43) Date de publication de la demande:
**15.05.85 Bulletin 85/20**

(45) Mention de la délivrance du brevet:
**07.12.88 Bulletin 88/49**

(84) Etats contractants désignés:
**CH DE FR GB IT LI NL**

(56) Documents cités:
**WO-A-81/00051**

(73) Titulaire: **FIRMENICH SA**
**1, route des Jeunes**
**CH-1211 Genève 8 (CH)**

(72) Inventeur: **Holzner, Günter**
**15, chemin des Palettes**
**CH-1212 Grand-Lancy (FR)**
Inventeur: **Morris, Anthony Francis**
**Villa "Lindos" 12, route Cantonale**
**CH-1261 Gingins (CH)**
Inventeur: **Rautenstrauch, Valentin, Dr.**
**69, chemin de Saule**
**CH-1233 Bernex (CH)**
Inventeur: **Thomas, Alan Francis, Dr.**
**Chemin de Grens**
**CH-1261 Borex (CH)**

(74) Mandataire: **Salvadori, Giuseppe, Dr.**
**c/o Firmenich S.A. Case Postale 239**
**CH-1211 Genève 8 (CH)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 141 266 B1**

## Description

La demande de brevet internationale publiée le 22 janvier 1981 sous le numéro WO 81/00051 décrit un article destiné au parfumage d'air ambiant ou d'enceintes fermées caractérisé en ce qu'il contient une composition parfumante enrichie en substances, odorantes ou pas, de faible polarité.

L'invention était basée sur l'observation que certaines substances pouvaient exercer un effet d'entraînement, au travers de feuilles polymériques, vis-à-vis des constituants polaires présents dans une composition parfumante donnée. Un tel principe, appliqué à la fabrication d'assainisseurs d'air ambiant par exemple, permettait la réalisation d'articles pouvant parfumer l'environnement de manière fidèle et équilibrée.

Parmi les substances pouvant exercer un tel effet d'entraînement figuraient notamment l'acétate de benzyle, l'acétate de linalyle, les terpènes en générale et les hydrocarbures d'utilisation courante en parfumerie, ainsi que les éthers dialkyl-glycoliques et les esters aromatiques ou aliphatiques de basse polarité. Nous avons maintenant découvert que parmi les substances terpéniques figure un éther dont le pouvoir "d'adjuvant d'entraînement" envers les autres ingrédients d'une composition parfumante à travers des membranes polymériques, était particulièrement marqué. Il s'agit de l'éther méthylique d'α-terpényle, composé chimique connu qui obéit à la formule suivante:

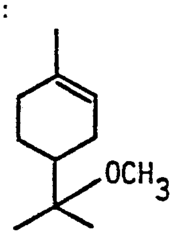

également connu sous le nom de 4-(2-méthoxy-2-propyl)-1-méthyl-1-cyclohexène.

L'effet parfumant exercé par ce composé sur des bases détergentes ou des savons a été décrit dans le brevet US no. 4,173,543 publié le 6 novembre 1979. Son emploi cependant en tant qu'activateur du pouvoir de diffusion d'une composition parfumante à travers des membranes polymériques était jusqu'ici inconnu et résulte d'une observation fortuite de notre part.

La présente invention a donc pour objet une telle utilisation particulière; elle est définie comme indiqué aux revendications.

L'éther méthylique de l'α-terpényle peut être obtenu par réaction du limonène avec de l'alcool méthylique en présence d'un catalyseur constitué par un acide protonique, organique ou minéral. La réaction s'effectue conformément aux techniques usuelles [voir à cet effet: Royals, J. Am. Chem. Soc., *71*, 2568-71, (1949)].

Il demeure entendu cependant que la réaction d'éthérification peut être effectuée non seulement sur le limonène pur, mais également sur des mélanges terpéniques le contenant. De tels mélanges constituent des matières premières qui sont abondamment disponibles dans l'industrie de la parfumerie et des arômes car ils proviennent en effet de l'extraction d'essence d'agrumes. Comme de tels mélanges s'obtiennent à partir de sources naturelles variées et résultent d'autre part de procédés parfois différents, leur composition ainsi que leur contenu en limonène peuvent varier. Nous avons pu déterminer que le produit obtenu de l'éthérification des résidus terpéniques résultant de l'extraction d'essence d'orange convenait parfaitement pour toute utilisation conforme à la présente invention.

Pour toute application pratique, le mélange terpénique tel qu'obtenu par distillation du mélange d'éthérification (fraction ayant Eb. 60—100°C/13,3 × $10^2$Pa) est utilisé de préférence. Leur contenu en l'éther désiré peut être de l'ordre de 50% ou plus, ledit éther étant accompagné par des quantités variables de limonène et d'autres dérivés éthérés monoterpéniques.

Des articles aptes à l'assainissement d'air ambiant constitués par des récipients en matière polymérique, ou comportant au moins une paroi en matière polymérique au travers de laquelle s'effectue la diffusion des vapeurs de substance active, ont été décrits dans la demande de brevet internationale WO 81/00051 mentionnée plus haut, ou dans la demande PCT/CH82/00005, publiée le 19 août 1982 souls le numéro WO 82/02700.

Les propriétés particulières de l'éther méthylique de l'α-terpényle ainsi que son efficacité seront illustrées à l'aide des essais comparatifs effectués par comparaison du produit en question avec d'autres des ingrédients courants utilisés jusqu'ici.

L'éther méthylique de l'α-terpényle peut être utilisé conformément à l'invention en tant que seul adjuvant de la composition assainissante active ou en mélange avec d'autres des substances "adjuvantes d'entraînement" connues, mentionnées notamment dans la demande de brevet internationale WO 81/00051. Par exemple, il peut être mélangé avec de l'acétate de linalyle, de l'acétate de benzyle ou des mélanges de terpènes.

Les proportions dans lesquelles l'éther de l'invention peut être utilisé pour promouvoir l'effet d'activateur de diffusion recherché, varient dans une gamme de valeurs assez étendue. De telles proportions peuvent être de l'ordre d'environ 10 à 90% en poids par rapport au poids total de la composition assainissante parfumée. Comme l'effet provoqué par l'emploi de l'éther méthylique de l'α-terpényle sur les autres constituants de la composition assainissante est d'en harmoniser la diffusion et de la rendre plus régulière dans le temps, les valeurs des proportions à utiliser seront donc fonction de la nature de ces autres constituants, et de la nature et l'épaisseur de la membrane polymérique au travers de laquelle ils doivent diffuser. L'expert pourra déterminer aisément ces valeurs par des essais comparatifs.

Les exemples qui suivent serviront à mieux

illustrer l'invention. Cependant, ils ne doivent pas être interprétés de façon limitative.

Exemple 1

Afin de tester le pouvoir de diffusion de l'éther méthylique de l'α-terpényle à travers des membranes polymériques, par comparaison avec celui de certains des adjuvants usuels, tels ceux mentionnés dans la demande de brevet internationale WO 81/00051, on a effectué les essais suivants.

Les substances volatiles à l'essai ont été posées entre deux feuilles d'éthylène-éthyl acrylate d'une épaisseur d'environ 0,1 mm et, au moyen de soudure, on a procédé à la manufacture de sachets d'une capacité d'environ 18 ml (appareils à soudure: Fermant 400, Joisten & Kettenbaum GmbH, Bensberg-Herkenrath, RFA). Le contenu de chacun des sachets obtenus était de 16 g de substance volatile. Les sachets ont été ensuite suspendus au moyen d'un crochet et maintenus dans une pièce normalement aérée à une température d'environ 20—25°C et leur poids a été déterminé à certains intervalles jusqu'à 56 jours.

La différence de poids observée entre les pesées détermine la mesure d'évaporation de la substance volatile.

Le diagramme montré à l'aide de la FIG 1 illustre le résultat de ces essais effectués à l'aide des substances volatiles suivantes:

a. mélange 50:50 (poids) d'éther méthylique de l'α-terpényle et d'acétate de benzyle;
b. mélange 50:50 (poids) d'éther méthylique de l'α-terpényle et d'acétate de linalyle;
c. acétate de benzyle;
d. acétate de linalyle;
e. éther méthylique de l'α-terpényle.

En analysant le diagramme mentionné, l'on peut observer que les substances volatiles a., b. et e. contenant l'éther méthylique de l'α-terpényle présentent une diffusion à travers les parois polymériques plus uniforme que celle des substances c. et d. ne contenant pas ledit éther.

D'autre part, en analysant la courbe de diffusion de b. avec celle montrée par d., l'on peut observer qu'après 56 jours la quantité résiduelle de b. était inférieure à celle de d.

Exemple 2

Un article plastique cylindrique du commerce à corps creux, utilisé en tant qu'axe des rouleaux de papier de toilette, et comportant dans sa surface latérale une série de fentes ou ouvertures aptes à permettre le passage des vapeurs de substances volatiles, a été utilisé en tant que support pour des dispositifs assainissants parfumés. De tels dispositifs ont été construits en forme de nacelle et comportent une paroi en matière polymérique constituée par une membrane d'éthylène-éthyl acrylate, perméable aux vapeurs des substances volatiles utilisées, ayant une surface d'environ 8 $cm^2$ et une capacité d'environ 12—15 cc.

Lesdites dispositifs ont été remplis à l'aide de compositions assainissantes constituées par des mélanges volatiles que voici (parties en poids):

I a. Caribic 812486DN (composition parfumante, origine: Firmenich SA, Genève), 30%
  b. éther méthylique de l'α-terpényle, 70%
II a. Caribic 812486DN, 30%
  b. terpènes Portugal, 70%
III a. Caribic 812486DN, 50%
  b. éther méthylique de l'α-terpényle, 35%
  c. terpènes Portugal, 15%

Le pouvoir de diffusion de ces différents mélanges au travers de la membrane polymérique de la nacelle a été mesurée par pesées successives à intervalles journaliers. Comme dans l'exemple précédent, la différence de poids observée entre les différentes pesées détermine le pouvoir d'évaporation de la substance volatile assainissante. Les résultats obtenus sont illustrés à l'aide du graphique montré à la FIG. 2.

On peut observer que les mélanges I et III diffusent de façon plus régulière dans le temps. Leur perte de poids initial est inférieure à celle de la composition II. Ce facteur est particulièrement important, car de ce fait, l'observateur perçoit un effet parfumant plus uniforme pendant toute la durée de l'utilisation du dispositif assainissant.

Exemple 3

En procédant de manière analogue à celle indiquée à l'exemple précédent, on a mesuré le pouvoir de diffusion de l'éther méthylique de l'α-terpényle par comparaison avec celui de l'acétate d'isobornyle. Les essais one été effectués à l'aide de deux dispositifs assainissants à nacelle de capacité différente, à savoir 12 et 10 cc comportant une paroi d'une surface de 8, respectivement 6 $cm^2$. Les résultats sont illustrés à l'aide des diagrammes montrés par les FIGS. 3 à 5.

L'éther méthylique de l'α-terpényle, utilisé dans les essais décrits ci-dessus, résultait d'un procédé d'éthérification d'un mélange terpénique provenant de l'extraction d'essence d'orange. Son contenu en l'éther indiqué était d'environ 50%. L'éther était accompagné d'autres produits terpéniques, tels le limonène et le terpinolène.

**Revendications**

1. Utilisation de l'éther méthylique de l'α-terpényle, ou de tout produit résultant de l'éthérification d'un mélange terpénique contenant des quantités prépondérantes de limonène à l'aide de méthanol en présence d'un catalyseur protique acide, à titre d'adjuvant à une composition assainissante parfumée.

2. Composition volatile parfumée, destinée à être utilisée dans des assainisseurs d'air ambiant ou d'enceintes fermées comportant au moins une paroi polymérique de diffusion, résultant de l'utilisation selon la revendication 1.

3. Article destiné à la diffusion de vapeurs assainissantes parfumées et comportant au moins une paroi polymérique de diffusion, caractérisé en ce qu'il contient en tant que substance active une composition selon la revendication 2.

4. Procédé pour la diffusion de vapeurs assainissantes parfumées caractérisé en ce qu'on

emploie comme moyen pour sa mise en oeuvre un article tel que défini à la revendication 3.

5. Composition volatile parfumée selon la revendication 2, caractérisée en ce qu'elle contient environ 10 à 90% en poids, par rapport au poids total de la composition, de l'éther méthylique de l'α-terpényle.

**Patentansprüche**

1. Verwendung von alpha-Terpenyl Methyl Äther oder Produkten, welche das Resultat der Verätherung eines Terpengemisches, das in vorwiegender Menge Limonen enthält, mit Methanol in Gegenwart einer protischen Säure sind, als Zusatz sur einer parfümierten sanierenden Komposition.

2. Eine flüchtige parfümierte Komposition resultierend aus der Verwendung nach Anspruch 1, dazu bestimmt als Raumluftbedufter oder Bedufter für geschlossene Räume, welcher zumindest aus einer polymeren Diffusionswand besteht, verwendet zu werden.

3. Zur Diffusion von parfümierten sanierenden Dämpfen bestimmter Gegenstand, welcher zumindest aus einer zur Diffusion geeigneten Polymerwand besteht, dadurch gekennzeichnet, dass er als aktive Substanz eine Komposition nach Anspruch 2 enthält.

4. Verfahren zur Diffusion von parfümierten sanierenden Dämpfen dadurch gekennzeichnet, dass man als Mittel zu seiner Realisierung einen Artikel, wie er im Anspruch 3 definiert ist, verwendet.

5. Eine flüchtige parfümierte Komposition nach Anspruch 2, dadurch gekennzeichnet, dass sie ungefähr 10—90% Gewichtsprozente, im Verhältnis zum Gesamtgewicht der Komposition, von alpha-Terpenyl Methyl Äthers enthält.

**Claims**

1. Utilization as an adjuvant to a perfumed sanitizing composition of alpha-terpenyl methyl ether or of any product resulting from the etherification with methanol in the presence of a protic acid catalyst of a terpene mixture containing predominant amounts of limonone.

2. Perfumed volatile composition, destined to be utilized in room fresheners or closed spaces having at least a polymeric wall for diffusion, resulting from the utilization according to claim 1.

3. Article destined to the diffusion of perfurmed sanitizing vapours having at least a polymeric wall for diffusion, characterized in that it contains as active substance a composition according to claim 2.

4. Process for the diffusion of perfumed sanitizing vapours characterized in that there is used an article as defined in claim 3.

5. Perfumed volatile composition according to claim 2, characterized in that it contains about 10 to 90% by weight, based on the total weight of the composition, of alpha-terpenyl methyl ether.

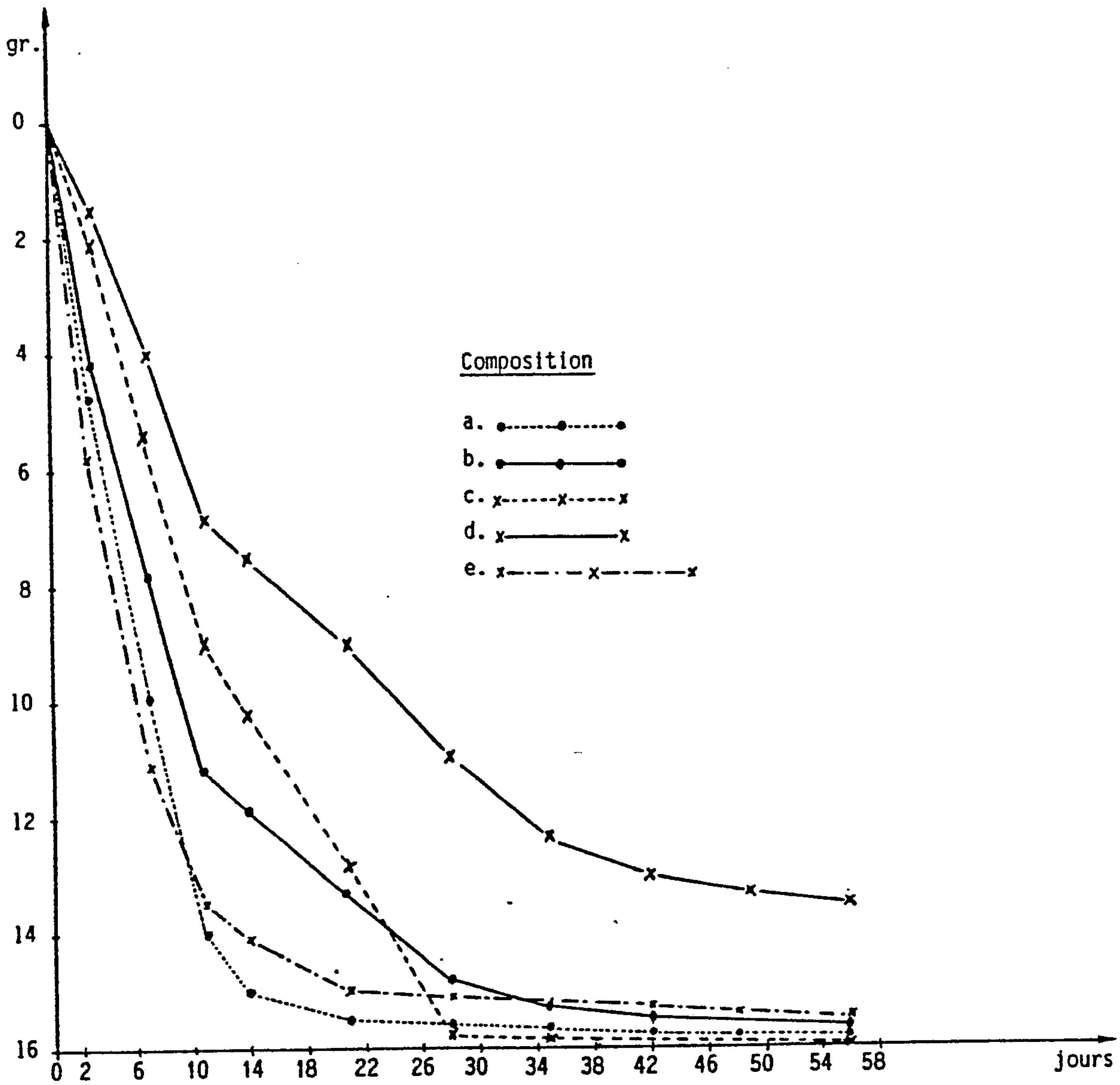
gr.
0
2
4
6
8
10
12
14
16
0 2 6 10 14 18 22 26 30 34 38 42 46 50 54 58
jours
Composition
a.
b.
c.
d.
e.

FIG 1

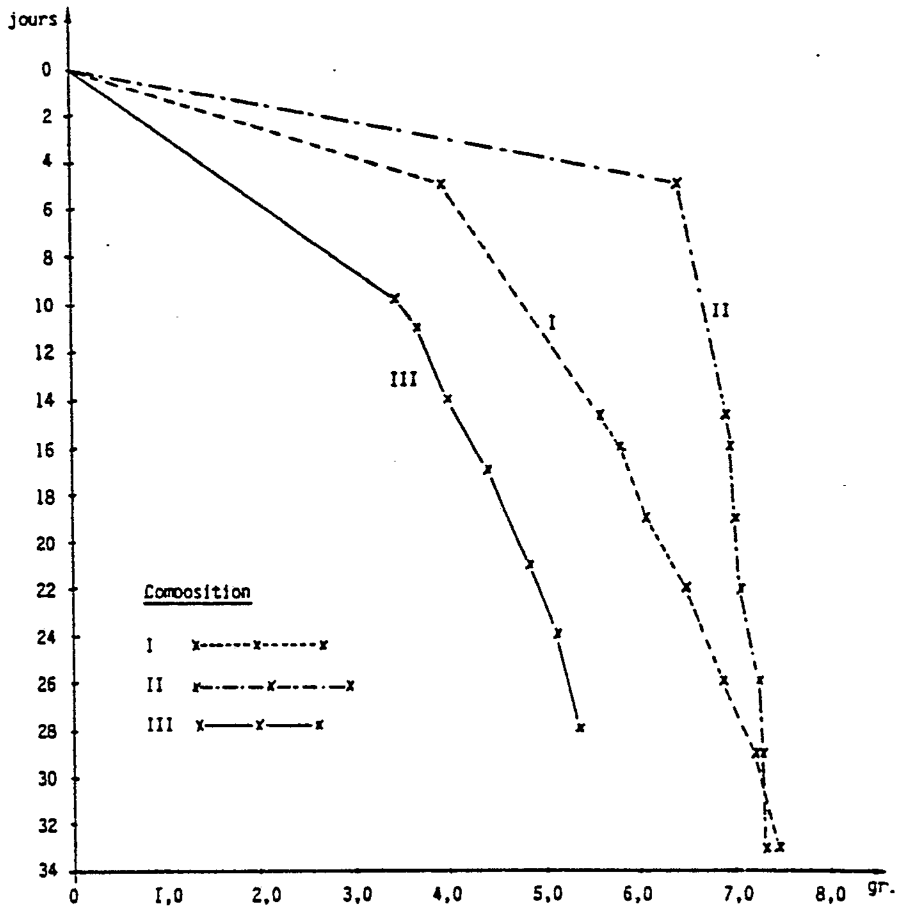
jours
0
2
4
6
8
10
12
14
16
18
20
22
24
26
28
30
32
34
0
I,0
2,0
3,0
4,0
5,0
6,0
7,0
8,0
gr.
I
II
III
Composition
I
II
III

FIG 2

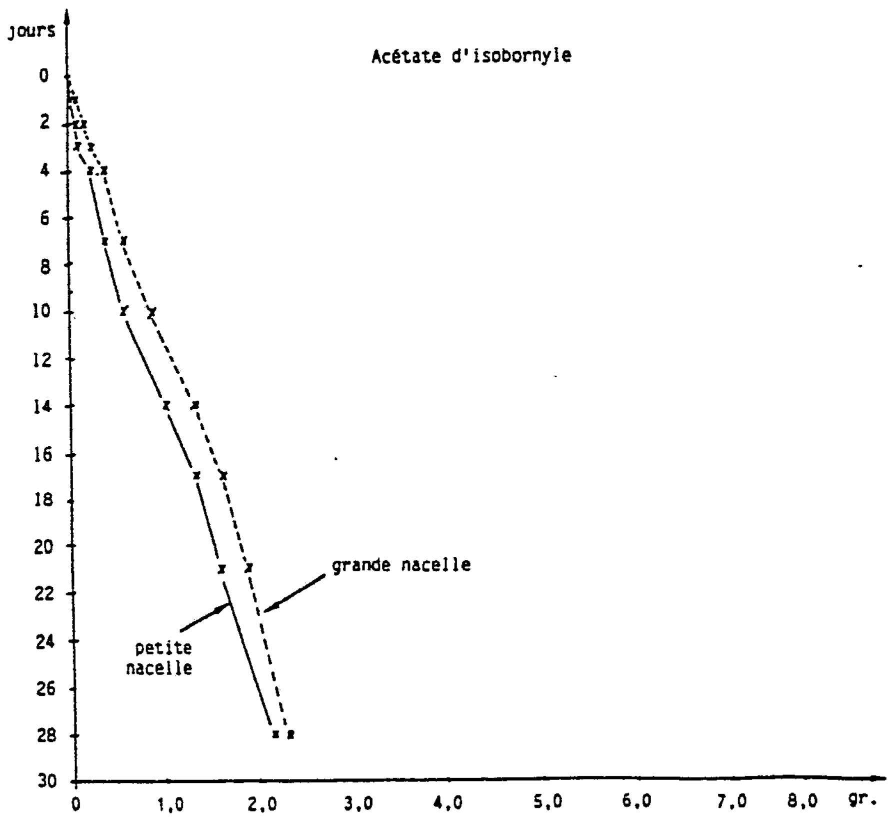
jours
Acétate d'isobornyle
0
2
4
6
8
10
12
14
16
18
20
22
24
26
28
30
0
1,0
2,0
3,0
4,0
5,0
6,0
7,0
8,0
gr.
grande nacelle
petite
nacelle

FIG 3

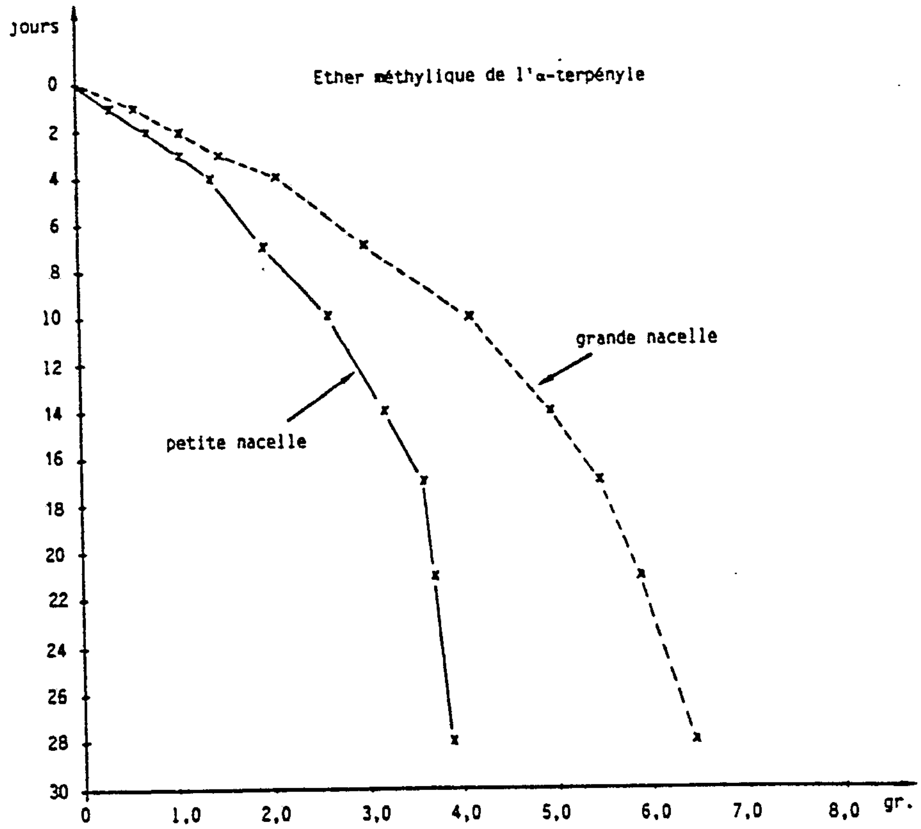
jours
Ether méthylique de l'α-terpényle
grande nacelle
petite nacelle
0
2
4
6
8
10
12
14
16
18
20
22
24
26
28
30
0
1,0
2,0
3,0
4,0
5,0
6,0
7,0
8,0
gr.

FIG 4

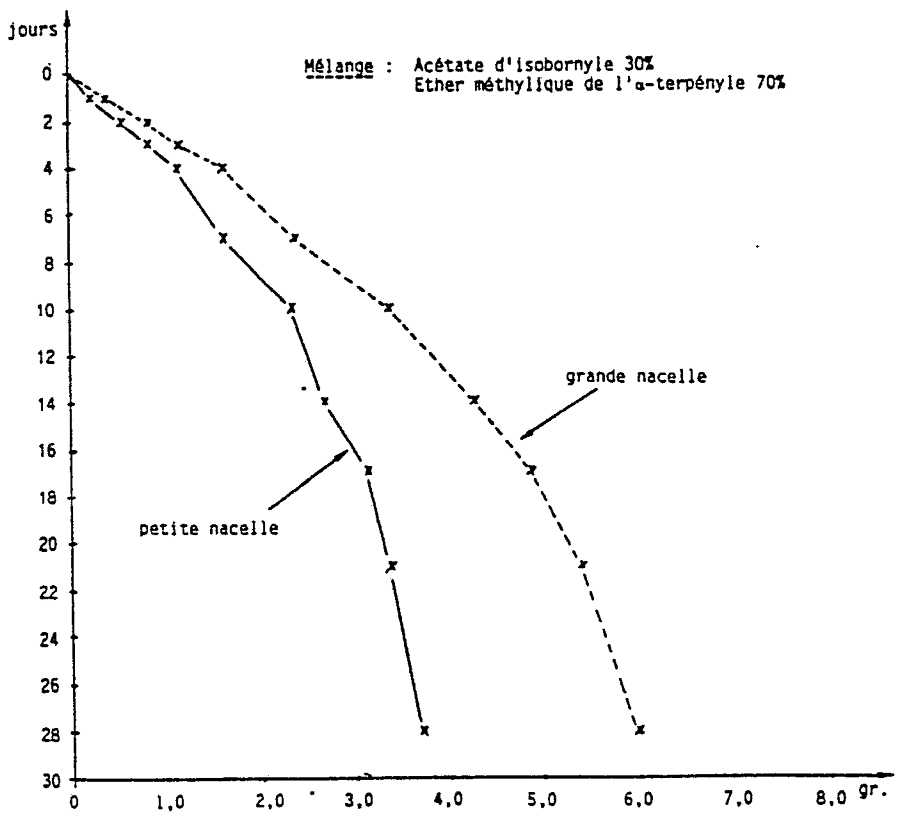
jours
Mélange : Acétate d'isobornyle 30%
Ether méthylique de l'α-terpényle 70%
grande nacelle
petite nacelle
0
2
4
6
8
10
12
14
16
18
20
22
24
26
28
30
0
1,0
2,0
3,0
4,0
5,0
6,0
7,0
8,0
gr.

FIG 5